# EUROPEAN PATENT APPLICATION

(11) **EP 4 483 933 A1**
(43) Date of publication of application: **01.01.2025**
(21) Application number: 23853682.5
(22) Date of filing: 15.05.2023
(51) Int. Cl.: A61M 16/04

(54) **CANNULA HOLDER**

(71) Applicant: Meilleur Co., Ltd., Chiba 274-0064 (JP)
(72) Inventor: YAMANE, Tsurashi, Chiba 274-0064 (JP)
(74) Representative: terpatent PartGmbB
(86) International application number: PCT/JP2023/018089
(87) International publication number: WO 2024/236688

(57) **Abstract**

Provided is a cannula holder capable of being used for various patients with different lengths around the necks. A cannula holder for securing a cannula to a neck of a patient with a tracheostomy includes a first belt and a second belt. The first belt includes: a long strip-shaped first body that has an inner surface and an outer surface; and a first hook-and-loop fastener that protrudes from a tip end of the first body in a longer-side direction and is inserted into a first slit of the cannula to be stuck on an outer surface of the cannula holder. The second belt includes: a long strip-shaped second body that is shorter than the first body and has an inner surface and an outer surface; a second hook-and-loop fastener that protrudes from a tip end of the second body in a longer-side direction and is inserted into a second slit of the cannula to be stuck on the outer surface of the second body; and a third hook-and-loop fastener that is secured within a range of the inner surface of the second body to be stuck on the outer surface of the first body.

## Description

### TECHNICAL FIELD

The present invention relates to a cannula holder used to secure a cannula to the neck of a patient.

### BACKGROUND ART

Conventionally, respiratory control is carried out by creating an opening in the trachea of a patient to insert a cannula therethrough and connecting a tube extending from a ventilator to the cannula. In such cases, a cannula holder is typically used to secure the cannula to the neck of a patient (see, for example, Patent Literature 1).

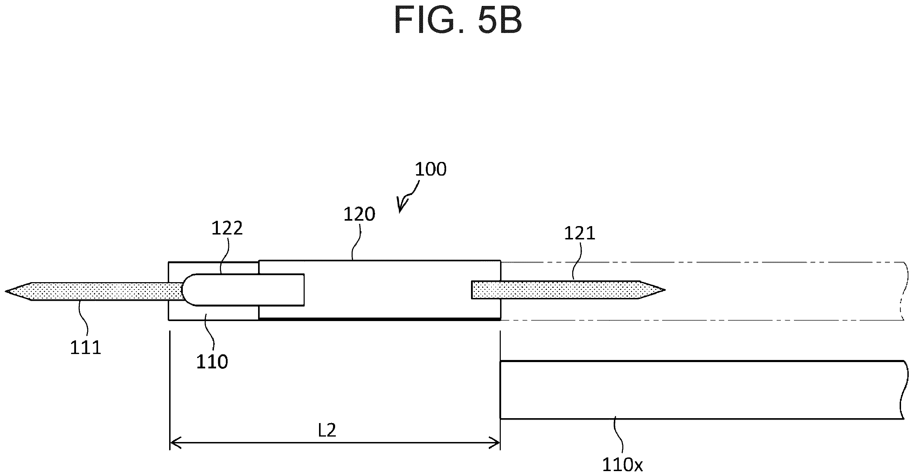

As illustrated in FIG. 5B, for example, a conventional cannula holder 100 includes a long belt 110 and a short belt 120. From one end in the longer-side direction of the long belt 110 and one end in the longer-side direction of the short belt 120, a tab 111 and a tab 121 to be connected to the cannula protrude, respectively. From the other end in the longer-side direction of the short belt 120, a hook-and-loop fastener 122 used to attach the short belt 120 to any position of the long belt 110 protrudes.

### CITATION LIST

### PATENT LITERATURE

Patent Literature 1: JP-A-2011-72379

### SUMMARY OF INVENTION

### TECHNICAL PROBLEM

Since different patients have different lengths around the necks, a cannula holder allowing its length to be adjusted for the patients is demanded. However, in the conventional cannula holder 100 with the hook-and-loop fastener 122 protruding from the other end in the longer-side direction of the short belt 120, its minimum length corresponds to the sum of the length of the short belt 120 and that of the hook-and-loop fastener 122 (=L2). This leads to a problem that it cannot be used for a patient whose length around the neck is extremely short, for example, for an extremely low birth weight baby.

The present invention has been made in view of the problem above, and an object thereof is to provide a cannula holder capable of being used for various patients with different lengths around the necks.

### SOLUTION TO PROBLEM

In order to solve the problem described above, the present invention provides a cannula holder for securing a cannula to a neck of a patient with a tracheostomy, comprising: a first belt; and a second belt, wherein the first belt includes: a long strip-shaped first body that has an inner surface and an outer surface; and a first hook-and-loop fastener that protrudes from a tip end of the first body in a longer-side direction, and is inserted into a first slit of the cannula to be stuck on an outer surface of the cannula holder, and the second belt includes: a long strip-shaped second body that is shorter than the first body and has an inner surface and an outer surface; a second hook-and-loop fastener that protrudes from a tip end of the second body in a longer-side direction, and is inserted into a second slit of the cannula to be stuck on the outer surface of the second body; and a third hook-and-loop fastener that is secured within a range of the inner surface of the second body to be stuck on the outer surface of the first body.

### ADVANTAGEOUS EFFECTS OF INVENTION

According to the present invention, it is possible to obtain a cannula holder capable of being used for various patients with different lengths around the necks.

### BRIEF DESCRIPTION OF DRAWINGS

[FIG. 1] FIG. 1 illustrates a state in which a cannula is secured to the neck of a patient.
[FIG. 2] FIG. 2 is an exploded perspective view of a cannula holder and a cannula.
[FIG. 3A] FIG. 3A illustrates an outer surface of a first belt.
[FIG. 3B] FIG. 3B illustrates an inner surface of a first belt.
[FIG. 4A] FIG. 4A illustrates an outer surface of a second belt.
[FIG. 4B] FIG. 4B illustrates an inner surface of a second belt.
[FIG. 5A] FIG. 5A illustrates a minimum length of a cannula holder according to the present embodiment.
[FIG. 5B] FIG. 5B illustrates a minimum length of a conventional cannula holder.
[FIG. 6] FIG. 6 illustrates a state in which a cannula holder is worn on an extremely low birth weight baby.
[FIG. 7] FIG. 7 is an enlarged view of a main portion of a cannula holder according to the first modification.
[FIG. 8A] FIG. 8A is a perspective view of a second body according to the second modification as viewed from an outer surface side.
[FIG. 8B] FIG. 8B is a perspective view of a second body according to the second modification as viewed from an inner surface side.

### DESCRIPTION OF EMBODIMENTS

Hereinafter, a cannula holder 1 according to an embodiment of the present invention will be described with reference to the drawings. The embodiments of the present invention described below are examples for realizing the present invention, and thus the scope of the present invention is not limited to the embodiment described herein. That is, the present invention can be realized with various modifications to be made to the embodiment described below.

### [Usage of Cannula Holder 1]

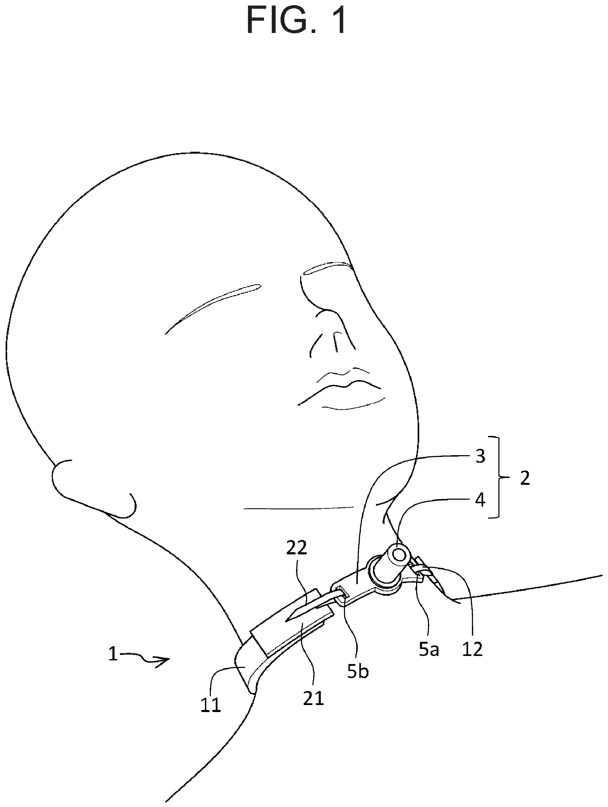

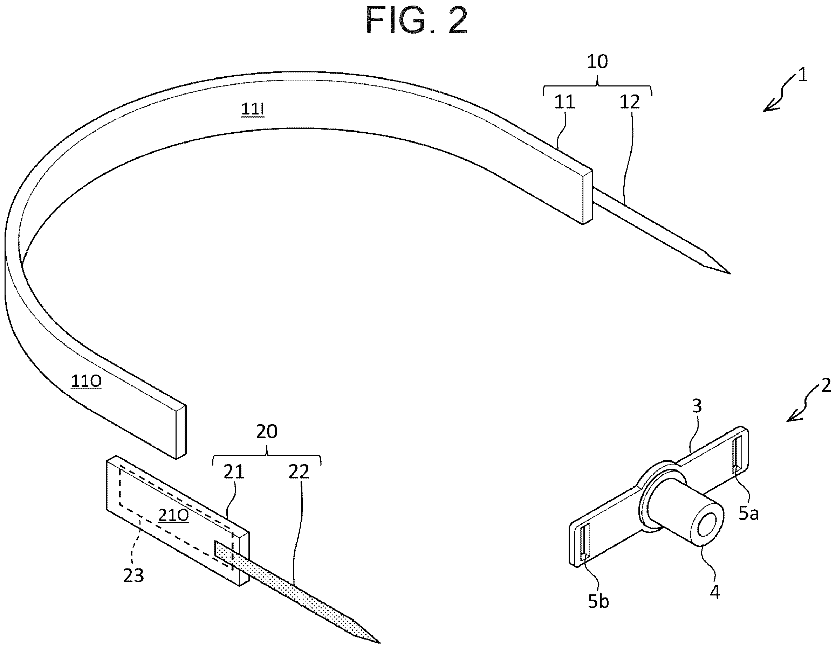

FIG. 1 illustrates a state in which a cannula 2 is secured to the neck of a patient. FIG. 2 is an exploded perspective view of the cannula holder 1 and the cannula 2. As illustrated in FIG. 1, the cannula holder 1 according to the present embodiment is used to secure the cannula 2 to the neck of a patient. The cannula holder 1 is detachable from the neck of the patient and from the cannula 2, which allows it to be repetitively used. Furthermore, the cannula holder 1 is adjustable in length so that it can be used for various patients with different lengths around the necks.

As illustrated in FIG. 1 and FIG. 2, the cannula 2 includes a body plate 3 and a tube 4. The body plate 3 has a long plate-like shape. On both the ends in the longer-side direction of the body plate 3, slits 5a, 5b that pierce the body plate 3 in the thickness direction are formed. The tube 4 pierces the body plate 3 in the thickness direction at the center in the longer-side direction (between the slit 5a and slit 5b) of the body plate 3.

As illustrated in FIG. 1, the cannula 2 is attached to an anterior region of the neck of a patient with a tracheostomy. One end (not illustrated) of the tube 4 protruding from the back surface of the body plate 3 is inserted into the opening that has been made in the trachea. On the other of the tube 4 protruding from the front surface of the body plate 3, a hose connected to a ventilator is to be connected. By means of the cannula holder 1, this cannula 2 is secured to the anterior region of the neck of the patient.

### [Structure of Cannula Holder 1]

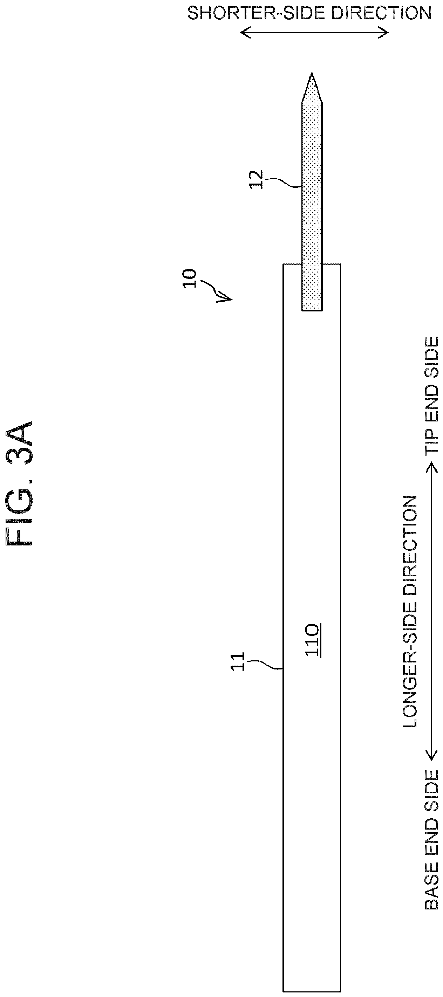

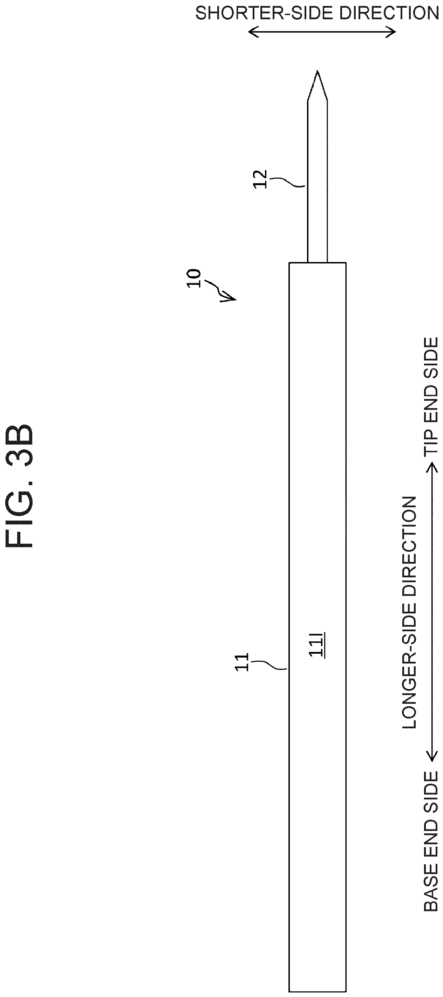

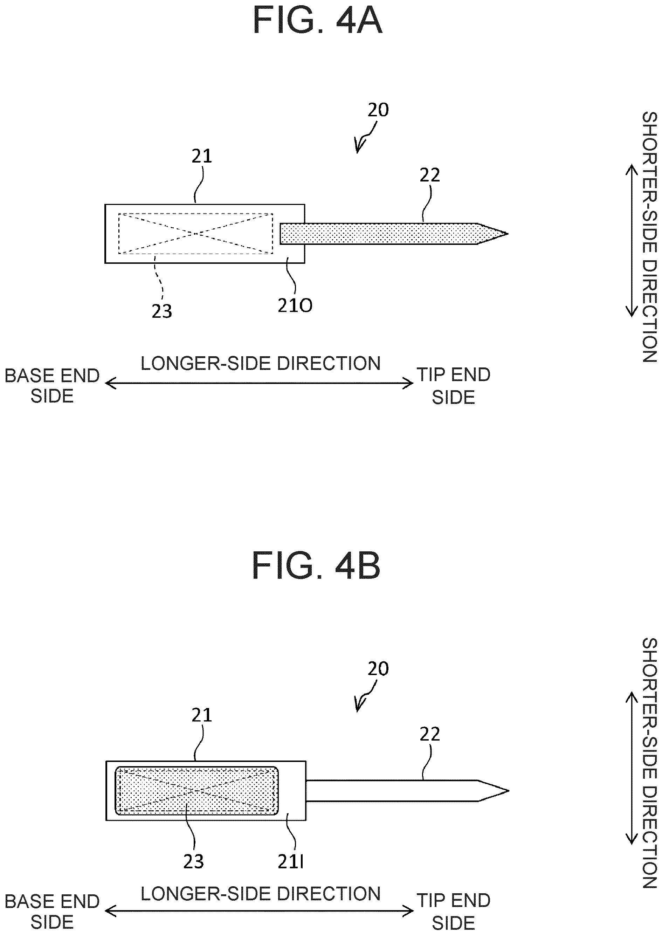

FIG. 3 illustrates an outer surface (A) and an inner surface (B) of a first belt 10. FIG. 4 illustrates an outer surface (A) and an inner surface (B) of a second belt 20. As illustrated in FIG. 1 to FIG. 4, the cannula holder 1 according to the present embodiment includes the first belt 10 and the second belt 20. As illustrated in FIG. 2 and FIG. 3, the first belt 10 includes a first body 11 and a first hook-and-loop fastener 12. As illustrated in FIG. 2 and FIG. 4, the second belt 20 includes a second body 21, a second hook-and-loop fastener 22, and a third hook-and-loop fastener 23.

Each of the first body 11 and the second body 21 is a long strip-shaped member, which has a substantially rectangular shape. The first body 11 and the second body 21 have inner surfaces 111, 21I and outer surfaces 11O, 210, respectively. The first body 11 and the second body 21 have flexibilities, which are thus bendable along the neck of a patient. The material used for the first body 11 and the second body 21 may be, for example, a fabric, a nonwoven fabric, a polyester, or a combination thereof.

The length in the longer-side direction of the second body 21 is less than the length in the longer-side direction of the first body 11. On the other hand, the length (width) in the shorter-side direction of each of the first body 11 and the second body 21 may be the same with each other, or the length in the shorter-side direction of the first body 11 may be slightly more than that of the second body 21. Hereinafter, for both the ends in the longer-side direction of the first body 11 and those of the second body 21, the ends on which the first hook-and-loop fastener 12 and the second hook-and-loop fastener 22 are provided will be referred to as "tip ends", and the ends on the opposite side will be referred to as "base ends".

The inner surface 11I of the first body 11 is made of a soft material (for example, a fabric) that gives less uncomfortableness to the patient when it touches his or her neck. The inner surface 21I of the second body 21 may be made of a similar material. On the other hand, each of the outer surfaces 11O, 210 of the first body 11 and second body 21 is made of a material (for example, a nonwoven fabric, a raised fabric, or a tail tape) that can serve as a female-side (loop surface) of each of the first hook-and-loop fastener 12, the second hook-and-loop fastener 22, and the third hook-and-loop fastener 23.

Each of the first hook-and-loop fastener 12, the second hook-and-loop fastener 22, and the third hook-and-loop fastener 23 is a male-side (a portion serving as a hook surface) of a so-called "magic tape (registered trademark)" or "Velcro (registered trademark)". In FIG. 2 to FIG. 5 and FIG. 7 to FIG. 8, the sides serving as the hook surfaces of the first hook-and-loop fastener 12, the second hook-and-loop fastener 22, and the third hook-and-loop fastener 23 are provided with hatching. The first hook-and-loop fastener 12, the second hook-and-loop fastener 22, and the third hook-and-loop fastener 23 may be attached to the first body 11 and the second body 21 by any method, for example, by welding, sewing, and the like.

Each of the first hook-and-loop fastener 12 and the second hook-and-loop fastener 22 has, for example, a long strip-shape. The first hook-and-loop fastener 12 and the second hook-and-loop fastener 22 are the portions for securing the cannula holder 1 to the cannula 2. The third hook-and-loop fastener 23 has, for example, a rectangular shape of which the vertexes are chamfered. The third hook-and-loop fastener 23 is the portion for securing the second body 21 to the outer surface 11O of the first body 11.

As illustrated in FIG. 3A, the first hook-and-loop fastener 12 is attached to the tip end of the outer surface 11O of the first body 11. In particular, the first hook-and-loop fastener 12 is attached to the center in the width direction of the first body 11. The first hook-and-loop fastener 12 protrudes in the longer-side direction of the first body 11 from the tip end. A hook surface of the first hook-and-loop fastener 12 faces in the same direction as the outer surface 11O of the first body 11 (in other words, an outer side in the state of the cannula holder 1 being wound around the neck of the patient). Then, as illustrated in FIG. 1, the first hook-and-loop fastener 12 is inserted into the slit 5a (first slit) of the cannula 2 from the inside, folded back, and stuck on the outer surface 11O of the first body 11 or the outer surface 210 of the second body 21.

As illustrated in FIG. 4A, the second hook-and-loop fastener 22 is attached to the tip end of the outer surface 210 of the second body 21. In particular, the second hook-and-loop fastener 22 is attached to the center in the width direction of the second body 21. The second hook-and-loop fastener 22 protrudes in the longer-side direction of the second body 21 from the tip end. A hook surface of the second hook-and-loop fastener 22 faces in the same direction as the outer surface 210 of the second body 21 (in other words, an outer side in the state of the cannula holder 1 being wound around the neck of the patient). Then, as illustrated in FIG. 1, the second hook-and-loop fastener 22 is inserted into the slit 5b (the second slit) of the cannula 2 from the inside, folded back, and stuck on the outer surface 210 of the second body 21.

As illustrated in FIG. 4B, the third hook-and-loop fastener 23 is attached to the inner surface 21I of the second body 21. In particular, the third hook-and-loop fastener 23 is attached within a range of the inner surface 21I of the second body 21. That is, in the plan view of the inner surface 21I of the second body 21, the third hook-and-loop fastener 23 does not protrude outward (especially, in the longer-side direction) from the outline of the second body 21. The hook surface of the third hook-and-loop fastener 23 faces in the same direction as the inner surface 21I of the second body 21 (in other words, an inner side in the state of the cannula holder 1 being wound around the neck of the patient). Then, the third hook-and-loop fastener 23 is stuck on any position of the outer surface 11O of the first body 11.

In the shorter-side direction of the first body 11 and second body 21, the width of the third hook-and-loop fastener 23 is more than the width of the first hook-and-loop fastener 12 and that of the second hook-and-loop fastener 22. In other words, in the shorter-side direction of the first body 11 and second body 21, the width of the first hook-and-loop fastener 12 and the width of the second hook-and-loop fastener 22 are less than the width of the third hook-and-loop fastener 23. Furthermore, in the shorter-side direction of the first body 11 and second body 21, the width of the first hook-and-loop fastener 12 and that of the second hook-and-loop fastener 22 are, preferably, sufficiently less than the width of the first body 11 and that of the second body 21 (for example, less than 1/2).

### [Minimum length of Cannula Holders 1, 100]

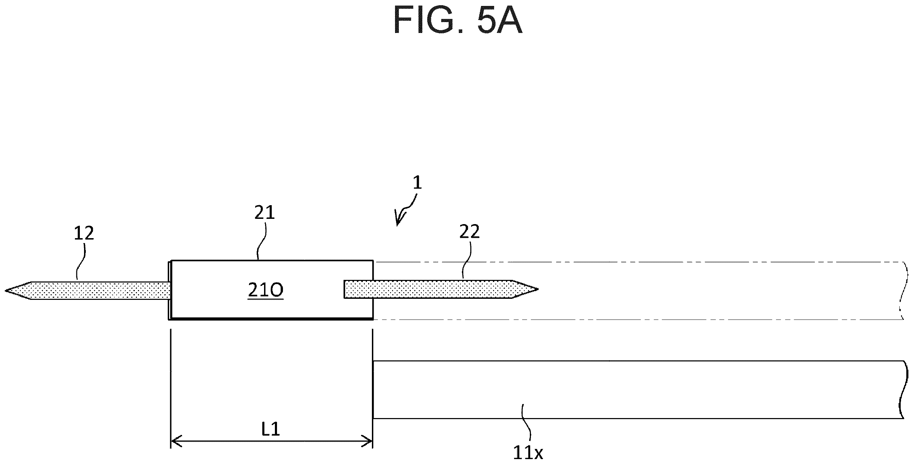

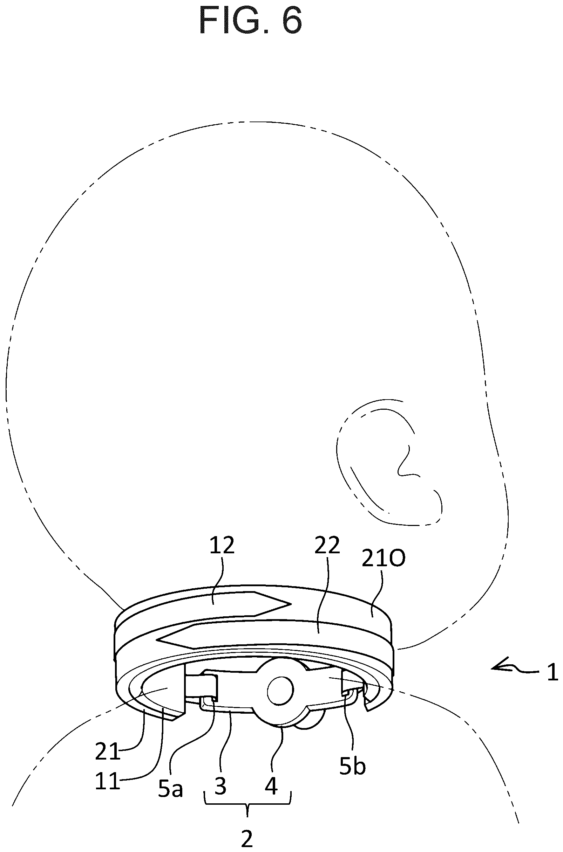

Next, referring to FIG. 5 and FIG. 6, the minimum length of the cannula holder 1 will be described. FIG. 5 illustrates a minimum length of the cannula holder 1 according to the present embodiment (A) and a minimum length of the conventional cannula holder 100. FIG. 6 illustrates a state in which the cannula holder 1 is worn on an extremely low birth weight baby.

As illustrated in FIG. 5A, in using the cannula holder 1 according to the present embodiment with its minimum length, the third hook-and-loop fastener 23 is stuck on the outer surface 11O of the first body 11 in a manner that the tip end of the first body 11 and the base end of the second body 21 are aligned with each other. In this state, the first body 11 and the second body 21 are attached with their tip ends being directed in mutually opposite directions. In the longer-side direction of the first body 11, an unnecessary portion 11x of the first body 11, which extends from the tip end of the second body 21 toward the base end of the first body 11 is to be cut off. At this time, the length of the portion of the cannula holder 1 to be wound around the neck of the patient (the minimum length) corresponds to a length L1 in the longer-side direction of the second body 21.

The first hook-and-loop fastener 12 and the second hook-and-loop fastener 22 protrude from the cannula holder 1 (the first body 11 and the second body 21 in their overlapping state) in the mutually opposite directions. Then, as illustrated in FIG. 6, the cannula holder 1 is wound around the neck of the patient, the first hook-and-loop fastener 12 inserted into the slit 5a and folded back is stuck on the outer surface 210 of the second body 21, and the second hook-and-loop fastener 22 inserted into the slit 5b and folded back is stuck on the outer surface 210 of the second body 21. Thus, the cannula 2 is secured to the neck of the extremely low birth weight baby. In this state, the first hook-and-loop fastener 12 and the second hook-and-loop fastener 22 are stuck on the outer surface 210 of the second body 21 at positions shifted in the shorter-side direction.

On the other hand, as illustrated in FIG. 1, for the case of securing the cannula 2 to the neck of an adult with the long length around the neck, the second body 21 is stuck on a portion of the first body 11 which is closer to the base end of the first body 11 as compared with the case illustrated in FIG. 5A. That is, in using the cannula holder 1 according to the present embodiment with its maximum length, the third hook-and-loop fastener 23 may be stuck on the outer surface 11O of the first body 11 in a manner that the base end of the first body 11 and the tip end of the second body 21 are aligned with each other.

In this case, the first hook-and-loop fastener 12 inserted into the slit 5a and folded back is stuck on the outer surface 11O of the first body 11, and the second hook-and-loop fastener 22 inserted into the slit 5b and folded back is stuck on the outer surface 210 of the second body 21. That is, the first hook-and-loop fastener 12 inserted into the slit 5a and folded back is stuck on either the outer surface 11O of the first body 11 or the outer surface 210 of the second body 21 (that is, the outer surface of the cannula holder 1) in accordance with an attachment position of the second body 21 with respect to the first body 11.

On the other hand, as illustrated in FIG. 5B, for the case of using the conventional cannula holder 100 with its minimum length, the hook-and-loop fastener 122 is stuck on the long belt 110 at a portion closer to the base end of the long belt 110 than the tip end of the long belt 110. Furthermore, in the longer-side direction of the long belt 110, an unnecessary portion 110x of the long belt 110, which extends from the tip end of the shorter belt 120 toward the base end of the long belt 110 is to be cut off. At this time, the length of the portion of the cannula holder 100 to be wound around the neck of the patient (minimum length) corresponds to a length L2 which is the sum of the length in the longer-side direction of the short belt 120 and that of the hook-and-loop fastener 122.

Thus, if the length in the longer-side direction of the short belt 120 and that of the second body 21 are the same, the minimum length L1 of the cannula holder 1 according to the present embodiment is less than the minimum length L2 of the conventional cannula holder 100 by the length of the hook-and-loop fastener 122 protruding from the short belt 120.

According to the embodiment described above, for example, the following advantageous effects can be obtained.

According to the embodiment described above, attaching the third hook-and-loop fastener 23 within a range of the inner surface 21I of the second body 21 enables the minimum length L1 of the cannula holder 1 to be shorten as compared with the case where the hook-and-loop fastener 122 protrudes from the base end of the short belt 120. Moreover, shifting the attachment position of the second body 21 toward the base end of the first body 11 enables the cannula holder 1 to be used for a patient with the length around the neck is long as well. Thus, the cannula holder 1 can be used for various patients with different lengths around the necks.

Furthermore, according to the embodiment described above, increasing the width of the third hook-and-loop fastener 23 more than the width of the first hook-and-loop fastener 12 and that of the second hook-and-loop fastener 22 enables the second body 21 to be stably attached to the first body 11. Moreover, reducing the width of the first hook-and-loop fastener 12 and that of the second hook-and-loop fastener 22 enables, as illustrated in FIG. 6, the first hook-and-loop fastener 12 and the second hook-and-loop fastener 22 to be shifted from each other in the width direction so as to prevent interference therebetween even in using the cannula holder 1 with its length being shortened.

### [First Modification]

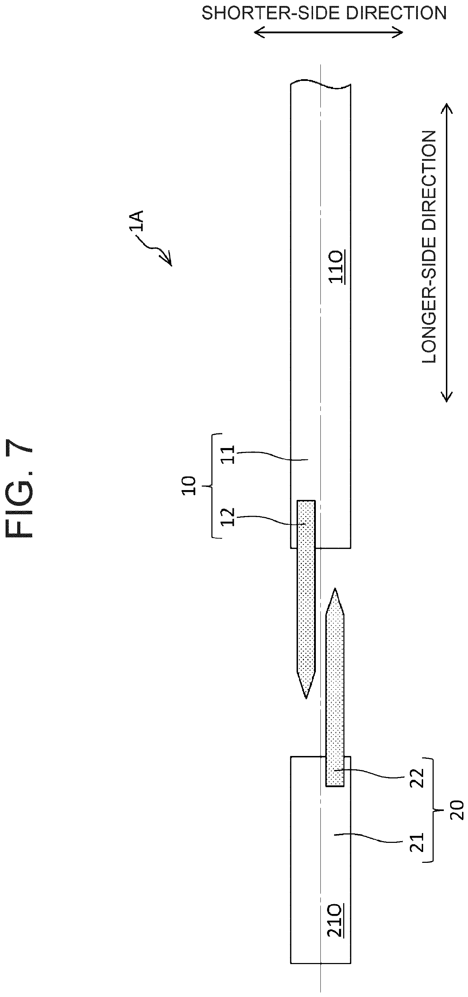

FIG. 7 is an enlarged view of a main portion of a cannula holder 1A according to the first modification. Hereinafter, the differences from the embodiment described above will be mainly described while the common features will not be described in detail. The cannula holder 1A according to the first modification differs from the embodiment described above in the attachment position of the first hook-and-loop fastener 12 with respect to the first body 11 and the attachment position of the second hook-and-loop fastener 22 with respect to the second body 21.

More specifically, the first hook-and-loop fastener 12 according to the first modification is arranged to be positioned closer to one side from the center (indicated with a chain line) in the shorter-side direction of the first body 11. The second hook-and-loop fastener 22 according to the first modification is arranged to be positioned closer to the other side from the center in the shorter-side direction of the second body 21. That is, in the cannula holder 1A wound around the neck of the patient, the first hook-and-loop fastener 12 and second hook-and-loop fastener 22 according to the first modification are arranged on the mutually opposite sides with the center in the shorter-side direction of the cannula holder 1A interposed therebetween.

According to the first modification, in the case as illustrated in FIG. 6, even without intentionally shifting the positions of the first hook-and-loop fastener 12 and second hook-and-loop fastener 22, the first hook-and-loop fastener 12 and the second hook-and-loop fastener 22 are stuck on the outer surface 210 of the second body 21 with being shifted from each other. This can prevent the first body 11 or the second body 21 from being twisted which is caused by shifting the positions of the first hook-and-loop fastener 12 and second hook-and-loop fastener 22, in using the cannula holder 1 with its length being shortened as illustrated in FIG. 6.

### [Second Modification]

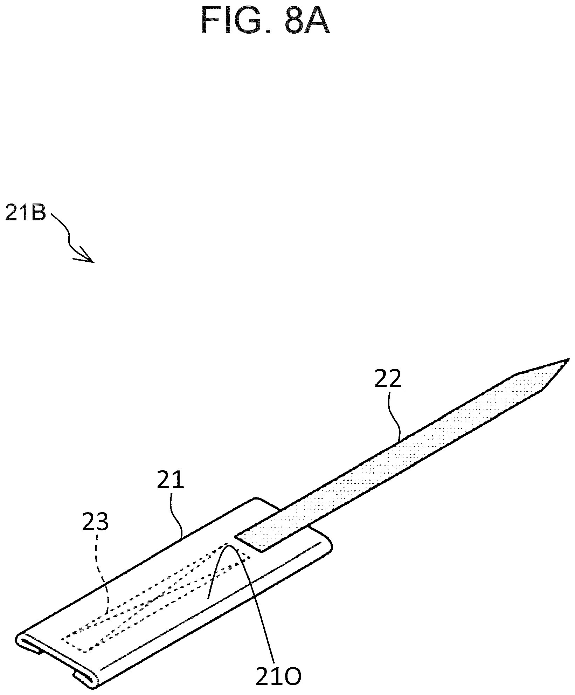

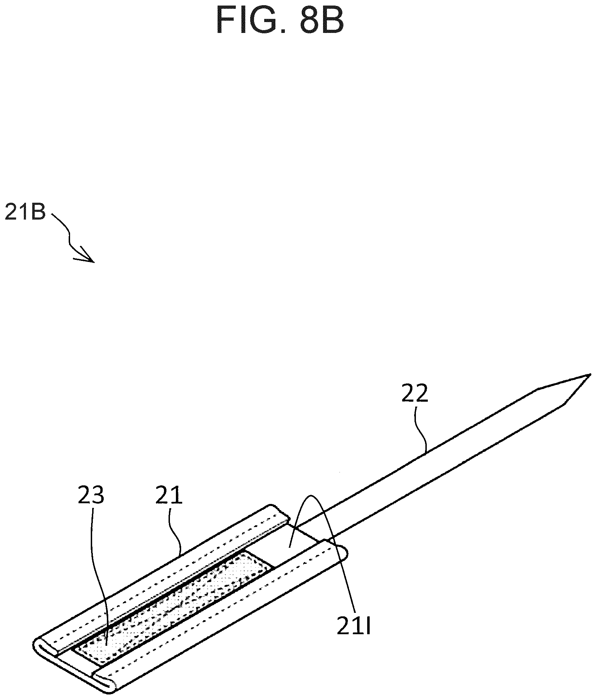

FIG. 8 is a perspective view of a second body 21B according to second modification as viewed from the outer surface side (A) and the inner surface side. Hereinafter, the differences from the embodiment described above will be mainly described while the common features will not be described in detail. The second body 21B according to the second modification differs from the embodiment described above in the shapes of both ends in the shorter-side direction thereof.

Both the ends in the shorter-side direction of the second body 21B according to the second modification are folded back toward the inner surface 21I of the second body 21B. The ends that have been folded back may be, for example, sewn or welded. Thus, in the second body 21B according to the second modification, each thickness of both the ends is thicker than that of the center portion in the shorter-side direction. Moreover, the width in the shorter-side direction of the second body 21B according to the second modification is less than the width in the shorter-side direction of the first body 11. However, using the flexibility of the second body 21B and bending the second body 21B so that the center portion in the shorter-side direction of the second body 21B protrudes allows the third hook-and-loop fastener 23 to be stuck on the outer surface 11O of the first body 11.

Repetitive use of the cannula holder 1 causes the width in the shorter-side direction of the first body 11 to gradually become thin. On the other hand, the width of the second body 21B in the shorter-side direction is maintained as well as new due to the third hook-and-loop fastener 23. This leads to the possibility that the third hook-and-loop fastener 23 protrudes from the first body 11 and directly touches the neck of the patient. However, according to the second modification, folding back both the ends in the shorter-side direction of the second body 21B enables the third hook-and-loop fastener 23 to be prevented from touching the neck of the patient even if the first body 11 becomes thin in the shorter-side direction.

Thus, the third hook-and-loop fastener 23 does not touch the neck, which prevents the patient from feeling discomfort. In this case, considering the possibility that the outer surface 210 of the second body 21B touches the neck of the patient, the outer surface 210 is preferably made of a soft material (for example, a raised fabric).

### REFERENCE SIGNS LIST

1, 1A ... Cannula holder, 2 ... Cannula, 3 ... Body plate, 4 ... Tube, 5a, 5b ... Slit, 10 ... First belt, 11 ... First body, 11I, 21I ... Inner surface, 11O, 210 ... Outer surface, 12 ... First hook-and-loop fastener, 20 ... Second belt, 21 ,21b ... Second body, 22 ... Second hook-and-loop fastener, 23 ... Third hook-and-loop fastener

## Claims

1. A cannula holder for securing a cannula to a neck of a patient with a tracheostomy, comprising:
a first belt; and
a second belt, wherein
the first belt includes:
a long strip-shaped first body that has an inner surface and an outer surface; and
a first hook-and-loop fastener that protrudes from a tip end of the first body in a longer-side direction, and is inserted into a first slit of the cannula to be stuck on an outer surface of the cannula holder, and
the second belt includes:
a long strip-shaped second body that is shorter than the first body and has an inner surface and an outer surface;
a second hook-and-loop fastener that protrudes from a tip end of the second body in a longer-side direction, and is inserted into a second slit of the cannula to be stuck on the outer surface of the second body; and
a third hook-and-loop fastener that is secured within a range of the inner surface of the second body to be stuck on the outer surface of the first body.

2. The cannula holder according to claim 1, wherein
in a shorter-side direction of the second body, a width of the third hook-and-loop fastener is more than a width of the second hook-and-loop fastener.

3. The cannula holder according to claim 1, wherein
both ends in a shorter-side direction of the second body are folded back toward the inner surface of the second body.

4. The cannula holder according to claim 1, wherein
the first hook-and-loop fastener is arranged to be positioned closer to one side from a center in a shorter-side direction of the first body, and
the second hook-and-loop fastener is arranged to be positioned closer to the other side from a center in a shorter-side direction of the second body.
